# EUROPÄISCHE PATENTANMELDUNG

(11) **EP 0 985 391 A2**
(43) Veröffentlichungstag der Anmeldung: **15.03.2000**
(21) Anmeldenummer: 99116456.7
(22) Anmeldetag: 21.08.1999
(51) Int. Cl.: A61F 13/02

(54) **Verbandmaterial auf Folienbasis**

(30) Priorität: 11.09.1998 DE 19841550
(71) Anmelder: Beiersdorf Aktiengesellschaft, 20245 Hamburg (DE)
(72) Erfinder: do Canto, Fabricio, 22999 Hamburg (DE)

(57) **Zusammenfassung**

Verbandmaterial auf Folienbasis, wobei die Folie aus Polyurethan besteht, wobei die Folie einseitig selbstklebend ausgerüstet ist und wobei an der Folie zumindest ein nicht klebender Anfasser angeformt ist, so daß das Verbandmaterial nach der Applizierung durch Ziehen am Anfasser in Richtung der Verklebungsebene vom Untergrund ablösbar ist.

## Beschreibung

Die Erfindung betrifft ein Verbandmaterial auf Folienbasis, das einseitig selbstklebend ausgerüstet ist und an dem zumindest ein nicht klebender Anfasser angeformt ist

Klebend beschichtete Trägersysteme, die auf Zug am Träger entkleben, sind bekannt. Sie basieren in der Regel auf elastischen Systemen, welche häufig Laminate darstellen.

DE-OS 27 28 346 beschreibt ein solches Klebeband, welches aus einer dehnfähigen Folie und einer Klebemasse auf Basis von A-B-A-Blockmischpolymerisaten besteht. Das gesamthaftende Laminat ist durch Strecken leicht von dem Untergrund zu lösen. Die Klebmasse ist vollflächig aufgetragen. Ein Anfasser zum Strecken des verklebten Bandes ist nicht vorgesehen.

DE-OS 195 31 696 beschreibt ein Klebfolienlaminat, welches aus einem dehnfähigen Träger und einer Acrylatklebemasse hergestellt wird.

WO 95/06691 offenbart ein wiederablösbares Klebeband, das einen Schaum als Trägermaterial aufweist.

WO 92/11333 beschreibt ein zerstörungsfrei entfernbares System, welches eine hohe Reißfestigkeit besitzt.

Die DE 40 26 755 A1 offenbart ein Verbandmaterial auf Folienbasis, das auf der einen Seite mit einem Stützmaterial abgedeckt ist, das die gleiche Größe wie die Folie besitzt und mindestens eine Griffleiste aufweist, und auf der anderen Seite mit einer selbstklebenden Schicht versehen ist. Die Griffleisten zur Entfernung des Trägermaterials sind innerhalb seiner Umfangsbegrenzung angeordnet. Vorzugsweise ist nur eine Griffleiste auf dem Trägermaterial angebracht.
Die DE 43 14 834 C2 beschreibt ein Verbandmaterial auf Folienbasis, das auf der einen Seite mit einem Trägermaterial abgedeckt ist, das die gleiche Größe wie die Folie besitzt und mindestens eine Griffleiste aufweist, und auf der anderen Seite mit einer selbstklebenden Schicht versehen ist. Die Griffleisten zur Entfernung des Trägermaterials sind innerhalb seiner Umfangsbegrenzung angeordnet. Vorzugsweise ist nur eine Griffleiste auf dem Trägermaterial angebracht.
Bei beiden der oben erwähnten Verbänden stellt sich nach der Applizierung das Problem, diese von der Hat abzulösen, weil diese vollflächig verklebt sind. Der Patient ist daher gezwungen, im Randbereich der Folie ein Stück derselben von der Haut abzuheben, indem er beispielsweise mit dem Fingernagel unter die Folie geht. Dieses Vorgehen ist zum einen sehr unbefriedigend, kann zum anderen insbesondere bei größeren Wunden und der damit einhergehenden gereizten Haut schmerzhaft sein.

Aus der EP 0 747 027 A2 ist ein Klebebandabschnitt bekannt, der aus einer Klebmasse besteht, die einseitig mit einer dünnen Abdeckfolie eingedeckt ist. Auf der gegenüberliegenden Seite sind zwei weitere Abschnitte einer Folie auf der Klebmasse vorhanden, so daß sich im Randbereich zwei Anfasser ergeben, mittel derer der Klebebandabschnitt durch Ziehen in Verklebungsebene vom Untergrund abgelöst werden kann.

Aufgabe der Erfindung ist es, die aus dem Stand der Technik bekannten Nachteile zu vermeiden und ein Verbandmaterial auf Folienbasis zur Verfügung zu stellen, das einseitig selbstklebend ausgerüstet ist und das sich durch Dehnung einfach und schmerzfrei nach der Applizierung auf der Haut wieder ablösen läßt.

Gelöst wird diese Aufgabe durch ein Verbandmaterial auf Folienbasis, wie es im Hauptanspruch niedergelegt ist. Gegenstand der Unteransprüche sind dabei vorteilhafte Weiterbildungen des Verbandmaterials.

Demgemäß wird erfindungsgemäß ein Verbandmaterial auf Folienbasis vorgeschlagen, wobei die Folie aus Polyurethan besteht, wobei die Folie einseitig selbstklebend ausgerüstet ist und wobei an der Folie zumindest ein nicht klebender Anfasser angeformt ist, so daß das Verbandmaterial nach der Applizierung durch Ziehen am Anfasser in Richtung der Verklebungsebene vom Untergrund ablösbar ist.

Die Verbandfolie selbst besteht aus elastischen, thermoplastischen Polyurethanen, wie sie in der DE-C 19 34 710 beschrieben sind und die sich durch eine gute Hautverträglichkeit sowie Sauerstoff- und Wasserdampfdurchlässigkeit auszeichnen. Besonders vorteilhaft haben sich aliphatische Polyesterurethane erwiesen.

Eine bevorzugte Folie ist etwa 30 bis 40 µm stark, transparent, weist eine Reißdehnung von über 450% und eine Wasserdampfdurchlässigkeit von über 500 g/m² in 24h bei 38 °C und 95% relative Feuchte nach DAB auf.

Die Dicke der Folie kann dabei zwischen etwa 15 bis 300, vorzugsweise 15 bis 80 µm, das Gewicht entsprechend zwischen etwa 15 bis 350 g/m², vorzugsweise 15 bis 100 g/m², die Höchstzugkraft längs zwischen etwa 5 bis 100 N/cm, vorzugsweise 2 bis 40 N/cm, und die Reißdehnung längs zwischen etwa 100 bis 1000% betragen.

Die Haftung der Folie auf dem Trägermaterial, die nur gering zu sein braucht (ca. 0,01 bis 0,5 N/cm, vorzugsweise 0,01 bis 0,05 N/cm), wird vorzugsweise dadurch bewirkt, daß die dünne Folie direkt auf dem Träger erzeugt wird durch Gießen, Rakeln, Extrudieren oder andere bekannte Methoden zur Filmherstellung. Falls notwendig, kann das Trägermaterial auf der Beschichtungsseite aufgerauht oder einer anderen haftungsfördernden Behandlung unterworfen werden. Auch eine adhäsionsfördernde Beschichtung kann vorteilhaft sein.

Wichtig dabei ist, daß die Haftung des fertigen Verbandes auf der Haut wesentlich stärker ist als die Haftung des Trägers an der Folie.

Die Anfasser sind insbesondere am Randbereich der Folie vorhanden, so daß die Herstellung des Verbandmaterials nicht weiter verkompliziert wird. Vorzugsweise ist nur ein Anfasser vorhanden. Durch Zug am Anfasser im wesentlichen in Richtung der Verklebungsebene wird die Folie gestreckt und löst sich gleichzeitig überraschend schmerzfrei von der darunterliegenden Haut.

Der Anfasser kann, um beim einzelnen gestanzten Pflaster möglichst wenig Siegelpapier beziehungsweise sonstiges Verpackungsmaterial einzusetzen, gefaltet werden, so daß er auf der Seite der Folie aufliegt, die nicht mit dem Kleber beschichtet ist.

In einer bevorzugten Ausführungsvariante ist die Folie auf der einen Seite mit einem Trägermaterial abgedeckt, das die gleiche Größe wie die Folie besitzt und mindestens eine Griffleiste aufweist.

Das Trägermaterial, das eine stützende Wirkung für die Folie hat, beim Aufbringen des Verbandes auf dieser verbleibt und erst nachträglich entfernt wird, besteht vorzugsweise aus einer Polyethylenfolie von etwa 80 µm Dicke, die auf ihrer Seite zur Folie hin leicht gerauht ist und dadurch matt, aber noch durchscheinend wirkt.

Es lassen sich auch andere Folien aus beispielsweise Polypropylen, Polyester, PVC oder geeignetes dünnes, beschichtetes Papier verwenden, sofern sie nur schmiegsam genug sind, um beim Anbringen des Verbandes nicht zu stören.

Ihre technischen Daten können sich in folgenden Bereichen bewegen:
- Dicke:: 30 - 300 µm
- Gewicht:: 30-350 g/m²
- Höchstzugkraft längs:: 5 - 100 N/cm
- Reißdehnung längs:: 10 - 3000%

Ihre Oberfläche zur Folie kann glatt, gerauht oder leicht geprägt sein.

Die Griffleisten bestehen vorzugsweise aus einer LDPE-Folie (low density polyethylene-Folie) von 80 µm Dicke. Um gut sichtbar zu sein, ist sie beispielsweise blau eingefärbt und wegen der besseren Griffigkeit an der Außenseite matt geprägt.

Anstelle dieser Folie können auch andere Materialien wie HDPE-, Polypropylen-, PVC-, PU-oder Potyesterfolien sowie Vliese, Papier oder Gewebe verwendet werden, sofern sie nur wieder ausreichend flexibel und anschmiegsam sind.

Die Dicke kann sich je nach Material zwischen 10 und 300 µm, das Gewicht entsprechend zwischen 10 bis 350 g/m² bewegen. Die Oberfläche kann matt, glänzend, rauh, glatt oder bedruckt sein. Die Höchstzugkraft längs kann je nach Material zwischen etwa 3 und 100 N/cm schwanken und die dabei auftretende Dehnung kann sich zwischen 5 und 500% bewegen.

Die Befestigung der Griffleisten an dem Trägermaterial kann je nach Material und Verarbeitungsmaschinen in verschiedenster Weise erfolgen, vorzugsweise durch Kleben oder Verschweißen. Das Kleben erfolgt dabei beispielsweise in der Weise, daß zwischen das Trägermaterial und den Griffleistenstreifen im zu verklebenden Bereich ein Streifen eines doppelseitig klebenden Klebebandes eingebracht wird oder eine Klebemassenbeschichtung aus einer Hotmelt-Masse oder einer Selbstklebemasse aus Lösungsmittel oder Dispersion.

Die Griffleisten können sich über die ganze Länge oder Breite des Verbandes erstrecken, weiterhin können sie bündig an mindestens einem Seitenrand abschließen.
Die Griffleisten können innerhalb der Umfassungsbegrenzung des Verbandmaterials angeordnet sein, sie können aber auch über den Rand des Verbandmaterials hinausstehen.
Vorzugsweise bestehen die Griffleisten aus einer LDPE-Folie.
Vorteilhafterweise ist nur eine Griffleiste auf dem Trägermaterial angebracht. Dann hat es sich als vorteilhaft erwiesen, wenn die Griffleiste in einem schmalen streifenförmigen Randbereich auf dem Trägermaterial befestigt ist und einseitig links oder rechts davon einen freien Anfaßbereich von mindestens etwa 5 mm aufweist beziehungsweise wenn die Griffleiste in einem mittigen schmalen, streifenförmigen Bereich auf dem Trägermaterial befestigt ist und zweiseitig davon einen freien Anfaßbereich von mindestens etwa 5 mm Breite aufweist.

Die Klebschicht auf der Folie weist beispielsweise bevorzugt eine Klebkraft auf Stahl von etwa 2 bis 4 N/cm auf, wobei das Prüfmaterial, da die Folie sehr dehnbar ist, für die Messung rückseitig mit einem unelastischen Klebefilm verstärkt werden muß. Die Messung selbst erfolgte in Anlehnung an DAB 9.

Das Verbandmaterial kann als solches verwendet werden, es kann jedoch auch zusätzlich mittig in geeigneter Breite eine übliche, saugende Wundauflage aufgebracht sein, welche kleiner als die Klebefläche ist, so daß es direkt als Wundverband eingesetzt werden kann.

Ein derartiger Verband mit Rundum-Verklebung ist besonders vorteilhaft, da er keimdicht und wasserfest ist.

Auf seiner selbstklebend ausgerüsteten, später der Haut zugewandten Seite ist das erfindungsgemäße Verbandmaterial über seine ganze Breite bis zum Gebrauch üblicherweise mit einem klebstoffabweisenden Trägermaterial, wie silikonisiertes Papier. abgedeckt. Dieses schützt die Selbstklebeschicht aus einer gut hautverträglichen Klebemasse, beispielsweise auf Acrylatbasis, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

In einer bevorzugten Ausführungsvariante weist das Verbandmaterial bei einer Zugbelastung von 10 N/cm eine Dehnung von größer 10% bis 3000% aufweist, besonders bevorzugt 20% bis 1000%.

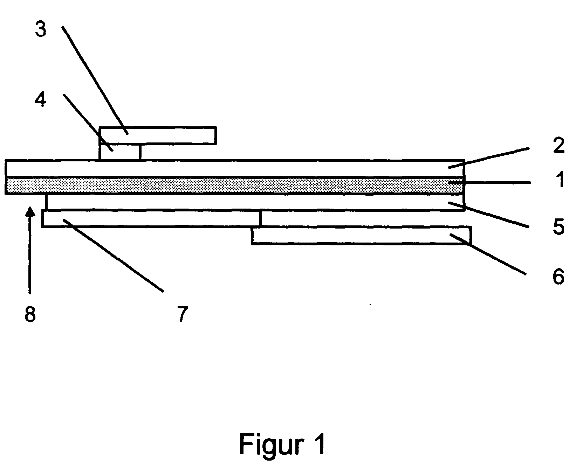

In der Figur 1 ist das erfindungsgemäße Verbandmaterial mit vergrößerten Dicken der Schichten beispielsweise dargestellt. Dabei bedeuten
- (1): die Folie,
- (2): den Träger,
- (3): die Griffleiste,
- (4): eine Klebeschicht zur Befestigung der Griffleiste am Träger,
- (5): die Selbstklebeschicht,
- (6) und (7): die Schutzabdeckungen für die Selbstklebeschicht und
- (8): der an der Folie angeformte Anfasser.

Im folgenden sollen anhand mehrerer Beispiele besonders vorteilhafte Ausführungsformen des Verbandmaterials dargestellt werden, ohne damit die Erfindung unnötig einschränken zu wollen.

### Beispiel 1

Das erfindungsgemäße Verbandmaterial besteht aus einer anionischen aliphatischen Polyesterpolyurethan-Dispersion-Folie (Impranil ® DLN Dispersion der Firma Bayer), und zwar in der Abmessung 50 x 50 mm. Die Folie weist eine Dicke von 0,1 mm auf.

Die Folie ist einseitig mit einer hautverträglichen Klebstoffschicht auf Basis vernetzter Polyacrylsäure-Derivate beschichtet.

Nach dem Verkleben soll das Verbandmaterial von der Haut entfernt werden, indem an einem an einer der Kanten der Folie vorhandenen und klebstofffreien Anfasser mit der Abmessung 10 x 50 mm gezogen wird.
Durch das Ziehen kommt es zum Entkleben der Folie, so daß das Verbandmaterial schmerzfrei angenommen werden kann.

### Beispiel 2

Das erfindungsgemäße Verbandmaterial wird analog zu Beispiel 1 von einem Polyurethangel 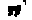 Cutinova thin" ® der Firma Beiersdorf AG gebildet, wobei dieser zusätzlich mit der Polyesterpolyurethan-Dispersion-Folie (Impranil ® DLN Dispersion der Firma Bayer) aus Beispiel 2 eingedeckt ist.

Auch bei diesem Wundverband ist ein schmerzfreies Entfernen nach dem Applizieren möglich, indem an einem an einer der Kanten der Folie vorhandenen und klebstofffreien Anfasser mit der Abmessung 10 x 50 mm gezogen wird.

## Patentansprüche

1. Verbandmaterial auf Folienbasis, wobei die Folie aus Polyurethan besteht, wobei die Folie einseitig selbstklebend ausgerüstet ist und wobei an der Folie zumindest ein nicht klebender Anfasser angeformt ist, so daß das Verbandmaterial nach der Applizierung durch Ziehen am Anfasser in Richtung der Verklebungsebene vom Untergrund ablösbar ist.

2. Verbandmaterial gemäß Anspruch 1, dadurch gekennzeichnet, daß die Folie auf der einen Seite mit einem Trägermaterial abgedeckt ist, das die gleiche Größe wie die Folie besitzt und mindestens eine Griftleiste aufweist.

3. Verbandmaterial gemäß Anspruch 1 und 2, dadurch gekennzeichnet, daß auf der klebenden Seite eine Wundaufiage angeordnet ist, welche kleiner als die Klebefläche ist.

4. Verbandmaterial gemäß Anspruch 1 bis 3, dadurch gekennzeichnet, daß die selbstklebende Seite mit einer abziehbaren Schutzabdeckung versehen ist.

5. Verbandmaterial gemäß Anspruch 1 bis 4, dadurch gekennzeichnet, daß die Folie bei einer Zugbelastung von 10 N/cm eine Dehnung von größer 10% bis 3000% aufweist, besonders bevorzugt 20% bis 1000%.
